(19) Europäisches Patentamt
European Patent Office
Office européen des brevets

(11) Numéro de publication: **0 003 717**
**B1**

(12) # FASCICULE DE BREVET EUROPEEN

(45) Date de publication du fascicule de brevet: **27.01.82**

(51) Int. Cl.³: **C 07 H 13/12**, A 23 K 1/22

(21) Numéro de dépôt: **79810007.9**

(22) Date de dépôt: **26.01.79**

(54) **Procédé de préparation et de purification de la lactosylurée.**

(30) Priorité: **14.02.78 CH 1585/78**

(43) Date de publication de la demande: **22.08.79 Bulletin 79/17**

(45) Mention de la délivrance du brevet: **27.01.82 Bulletin 82/4**

(84) Etats contractants désignés: **BE CH DE FR GB IT LU NL SE**

(56) Documents cités:
**DE-C-727 691**
**GB-A-1 515 280**
**US-A-2 612 497**
**US-A-3 969 846**
**US-A-4 066 750**

(73) Titulaire: **BATTELLE MEMORIAL INSTITUTE, 7 route de Drize, CH-1227 Carouge/Genève (CH)**
(84) Etats contractants désignés: **GB IT SE**

(73) Titulaire: **SOLAGRO S.A., Rue du Prince Albert 33, B-1050 Bruxelles (BE)**
(84) Etats contractants désignés: **BE CH DE FR LU NL**

(72) Inventeur: **Sachetto, Jean-Pierre, L'Escalade rue de Savoie 3, F-74160 St Julien-en-Genevois (FR)**
Inventeur: **Cuccolo, Sergio, Rue Alcide Jentzer 9, CH-1205 Genève (CH)**
Inventeur: **Regnault, Alain, Villard-Tacon par Ornex, F-01210 Ferney-Voltaire (FR)**
Inventeur: **Armanet, Jean-Michel, rue des Bossons 8, CH-1213 Onex (CH)**
Inventeur: **Tournier, Hervé-Noel, Le Riondet, F-74520 Valleiry (FR)**

(74) Mandataire: **Dousse, Blasco et al, 7, route de Drize, CH-1227 Carouge/Genève (CH)**

**EP 0 003 717 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Procédé de préparation et de purification de la lactosylurée

La présente invention concerne un procédé de préparation et de purification de la lactosylurée, ce composé est utile comme ingrédient de fourrage pour animaux.

On sait que les ruminants sont capables, tout au moins partiellement, d'assimiler l'azote des matières non protéiques telles que l'urée et les sels d'ammonium. Ainsi, pour économiser les aliments végétaux, on donne souvent au bétail du fourrage additionné d'urée libre. Celle-ci se décompose enzymatiquement dans la panse de ces animaux en libérant de l'ammoniac, ce composé étant utilisé par les micro-organismes du tube digestif, conjointement aux autres produits de la digestion, pour la synthèse des acides aminés nécessaires à la constitution des tissus vivants. Or, la présence d'urée libre peut parfois causer, dans l'estomac des animaux, la formation d'un excès momentané d'ammoniac avec pour conséquences des risques d'intoxication de ceux-ci et une perte notable d'azote par excrétion.

Cependant, on a constaté qu'il était possible de remédier à ces inconvénients en utilisant, à la place d'urée libre, des produits de condensation de celle-ci avec des glucides, par exemple la glucosylurée et la xylosylurée, lesquels présentent de notables avantages. Ainsi, dans le cas de fourrage contenant, par exemple, de la glucosylurée, l'hydrolyse enzymatique de ce composé et la libération de $NH_3$ sont beaucoup plus lents et, indépendamment de la suppression des risques d'intoxication, l'assimilation des aliments et l'utilisation de cet ammoniac libre sont notablement améliorés. On trouvera, dans la demande de brevet No 7159/77 de la titulaire un bref résumé de l'art antérieur concernant un tel sujet.

Ainsi, on a préconisé dans le passé l'utilisation de plusieurs glycosylurées comme ingrédients complémentaires de fourrages, par exemple les produits de condensation acide entre l'urée et les mélasses ou les produits d'hydrolyse du bois et de la cellulose. Récemment, (Proc. Nat. Soc. 34 (1975), 90 A; Brevets USP 3.677.767 (CARGILL) et 4.066.750 (ASTRA)) on a proposé dans ce but, la lactosylurée dont la préparation a été signalée dans les références suivantes: Rec. Trav. Chim. 22 (1903), 72—77 et Berichte 59B (1926) 69. Le procédé de préparation consiste, selon une des formes d'exécution, à chauffer plusieurs jours le lactose, soit cristallisé, soit sous forme de précipité du petit lait par la présure, avec de l'urée en solution aqueuse en présence d'un acide minéral, par exemple $H_2SO_4$. Suivant cet art antérieur, on chauffe à des températures ne dépassant pas 60°C pour éviter les risques de décomposition du disaccharide en hexose. Une telle décomposition a d'ailleurs été signalée dans le brevet américain USP 2,612,497 lequel indique que pour former des uréido-hexoses dans telles conditions (par exemple entre 60 et 100°C) on peut partir de disaccharides comme le lactose et le maltose lesquels s'hydrolysent en monosaccharides avant de se condenser avec l'urée.

Suivant une autre forme d'exécution (USP 4.066.750), on chauffe à 70°C 15 heures une solution aqueuse diluée de lactose (40—50% en poids) et d'urée avec une proportion relativement élevée d'acide minéral (environ 10% en poids) ce qui fournit des taux de conversion lactose → lactosylurée atteignant 85%. Cependant, cette méthode ne se prête pas à l'isolation directe de lactosylurée de haut degré de pureté car la solution acide doit être d'abord neutralisée puis concentrée pour faire précipiter de la lactosylurée impure qu'on recristallise ensuite, par exemple dans de l'eau.

Or, on a maintenant découvert avec étonnement qu'en travaillant en présence de faibles quantités d'eau et d'acide, on peut obtenir la lactosylurée avec un excellent rendement à des températures supérieures à 70° ce qui permet de réduire considérablement le temps réactionnel et est bien plus économique.

Ainsi, le procédé de l'invention dans lequel on chauffe le lactose et l'urée en présence d'eau et d'un catalyseur acide est caractérisé par le fait qu'on opère à une température d'au moins 70°C et ne dépassant pas 110°C avec une quantité d'eau minimum quoique suffisante pour maintenir le mélange fluide (tout au moins en début de réaction) mais, cependant, assez consistant pour qu'on puisse le déposer sur une surface horizontale sans qu'il se mette à couler. De plus, il peut être avantageux d'opérer de manière que cette eau s'évapore progressivement au cours de la réaction, ceci étant valable notamment s'il n'est pas jugé indispensable d'éliminer de la lactosylurée obtenue en fin de réaction les résidus d'urée et de lactose n'ayant pas réagit ainsi que le catalyseur acide.

En procédant de cette manière, on peut facilement atteindre des rendements de l'ordre de 85% après seulement 2 heures à 110°C, ce chiffre pouvant être encore notablement augmenté par un chauffage plus prolongé ou en travaillant sous pression réduite, de manière à accélérer le départ de l'eau réactionnelle. Il est d'ailleurs à remarquer que la température de travail optimum dépend du catalyseur utilisé comme on le verra plus loin.

Comme lactose de départ on peut utiliser du lactose cristallisé ou du lactose monohydrate brut obtenu par concentration du lactoserum déprotéinisé, un produit très bon marché.

Comme catalyseur acide, on peut utiliser dans le présent procédé, les catalyseurs usuels de la condensation de l'urée avec les sucres et, notamment, $H_2SO_4$, $H_3PO_4$, HCl, $H_2NSO_3H$, etc... On préfère utiliser $H_2SO_4$ en quantité d'environ 0,5—2% par rapport au poids du mélange réactionnel. De plus, il est avantageux d'ajouter le catalyseur acide par portions, progressivement au cours de la réaction, ceci de manière à compenser la neutralisation d'une partie de cet acide par l'ammoniac éventuellement libéré suivant la réaction:

$$H_2N-CO-NH_2 + H_2O \longrightarrow 2NH_3 + CO_2$$

En effet, on a constaté qu'aux températures du haut de la gamme précitée, il existe une certaine tendance à la décomposition des sels formés entre l'urée et l'acide utilisé comme catalyseur; cette tendance dépend d'ailleurs de la nature de l'acide dont dérive un tel sel. Ainsi, par exemple, le sulfate d'urée n'a tendance à se décomposer de façon notable qu'au-dessus de 90°C tandis que le phosphate d'urée, moins stable, se décompose déjà aux alentours de 75°C. La température optimum sera donc celle où le compromis le plus favorable sera réalisé entre la vitesse de réaction qui augmente avec la température et la décomposition des sels d'urée qui nécessitent l'apport d'une quantité nouvelle de catalyseur dont on désire, autant que possible, éviter l'excès. Dans le cas d'utilisation, comme catalyseur, de $H_2SO_4$, l'intervalle de température qu'on est de 75 à 85°.

Il faut encore noter que les considérations qui précèdent concernant la stabilité comparée des sels d'urée à diverses températures ne s'appliquent, en valeurs absolues, que pour une concentration d'eau donnée du mélange réactionnel. Si la quantité d'eau diminue, la tendance à la décomposition à une certaine température diminue en raison de la loi d'action des masses. En conséquence, si l'eau s'évapore rapidement au cours de la réaction de condensation, la tendance à la décomposition de l'urée perd son importance et les températures de chauffe pourront être plus élevées avec des temps réactionnels plus courts.

La quantité d'eau à ajouter au mélange réactionnel est aussi faible que possible, compte tenu que la masse doit être maintenue suffisament fluide pour être agitée, au moins pendant les premiers temps de réaction; de préférence, pour un rendement optimum, la quantité d'eau ne devrait pas dépasser environ 20 à 25% en poids du mélange réactionnel.

Il est à remarquer par ailleurs que les produits de condensation acide entre l'urée et les saccharides déjà connus et utilisés comme fourrage sont des mélanges, généralement simplement neutralisés, contenant de fortes proportions d'urée et de sucres n'ayant pas réagi. Or, la titulaire a trouvé, comme elle l'a indiqué dans la demande de brevet No 7159/77, que les glycosylurées cristallisées purifiées de leur urée libre présentaient, pour l'alimentation du bétail, de grands avantages par rapport aux mélanges de produits de condensation susmentionnés. Ainsi, par exemple, la glucosylurée de synthèse, une fois débarrassée de l'urée libre qu'elle contient à l'état brut, a non seulement le pouvoir de libérer son ammoniaque à un taux très modéré, mais elle favorise encore de façon surprenante la digestion et l'assimilation des produits cellulosiques ordinaires ingérés en même temps qu'elle. L'adjonction de glycosylurées purifiées aux aliments pour bétail riches en cellulose poursuit donc un double but:

Retarder la libération de l'ammoniaque de l'urée et ainsi favoriser son assimilation par l'animal et, simultanément, améliorer la digestion des produits cellulosiques présents dans ces aliments.

Ainsi, on a trouvé qu'on pouvait purifier la lactosylurée préparée par le procédé susmentionné et éliminer l'urée résiduelle non transformée jusqu'à un taux ne dépassant pas 2,5% en traitant la masse réactionelle par l'eau ou un solvant hydrophile et en séparant le solide insoluble purifié du liquide renfermant les impuretés. Ainsi, suivant une première forme d'exécution, après refroidissement de la masse réactionelle brute, on broie celle-ci et la triture avec de l'éthanol, puis on sépare la lactosylurée ainsi purifiée de l'éthanol par essorage.

L'éthanol possède en effect la propriété inattendue de dissoudre sélectivement l'urée non transformée à l'exception d'une petite quantité de celle-ci qui reste fixée à l'acide catalyseur en tant que sel d'urée. Ein général ce taux résiduel d'urée est inférieur à 2,5%.

Suivant une autre forme d'exécution, on reprend la masse réactionelle brute (de préférence lorsqu'elle est encore chaude et bien fluide) et on la verse sur de la glace ou de l'eau glacée, de manière qu'après ce traitement la température du mélange soit de l'ordre de 5 à 30°C, puis on essore ou on centrifuge le solide non dissous, les impuretés à éliminer (c'est-à-dire l'acide catalyseur, l'urée libre et une partie du lactose n'ayant pas réagi) étant entraînées par les eaux-mères. Il est à remarquer que cette méthode de purification permettant d'éliminer pratiquement complètement l'urée n'ayant pas réagi, il peut être avantageux, pour améliorer les rendements de condensation entre le lactose et l'urée, d'utiliser, au départ, un excès de celle-ci par rapport au lactose. Bien entendu, dans ce cas, on récupère ensuite l'urée en excès, par concentration et cristallisation des eaux-mères de lavage suivant les moyens habituels.

La lactosylurée peut être ajoutée au fourrage des animaux sous forme cristallisée (dispersion dans la paille ou le foin) ou sous forme de solution aqueuse. Elle peut être également mélangée à des tourteaux ou à des céréales (orge, maïs, blé). Comme telle, elle présente déjà des propriétés très intéressantes en tant que libération lente de l'azote combiné mais cette progressivité peut encore être améliorée en la convertissant en son dérivé N-méthylol par l'action du formaldéhyde.

On peut effectuer cette réaction par les moyens usuels c'est-à-dire en dissolvant la lactosylurée dans du formol à 30% avec addition de soude caustique jusqu'à alcalinisation, chauffage quelques heures puis concentration jusqu'à cristallisation.

Les exemples qui suivent illustrent l'invention.

Exemple 1

Dans un réacteur cylindrique de 1 litre à large ouverture, doté d'un puissant agitateur, on mélange 36 g de lactose monohydrate (0,1 mole) et 10 ml d'eau. On porte la température du mélange à 70° C. Après 10 minutes on ajoute 60 g d'urée (1 mole) sous agitation et l'on obtient une masse pâteuse pouvant être agitée. Après avoir bien homogénéisé cette masse, on additionne sous agitation, en maintenant le mélange à 70° C, 324 g de lactose monohydrate (0,9 mole) par fractions de 36 g (soit 9 fractions), c'est-à-dire une fraction toutes les 10 minutes. Après chaque fraction de lactose, on ajoute un minimum d'eau pour pouvoir maintenir l'agitation, le mélange restant à l'état pâteux. Pendant les 90 minutes que dure cette addition de lactose, on ne devrait pas dépasser un total de 50 ml d'eau, additionnés par portions de quelques ml. Après avoir additionné tout le lactose, on ajoute 3 ml (5,5 g) de $H_2SO_4$ 98% dans 10 ml d'eau et l'on maintient le mélange à 70° C en agitant aussi longtemps que possible. Après 8 heures, on rajoute encore 20 ml d'eau, car le mélange e tendance à se prendre, et l'on poursuit le chauffage à 70° C. Après 10 heures, on peut arrêter l'agitation et en fin de réaction, après 23 heures, on obtient une masse blache suffisamment friable pour qu'on puisse la sortir facilement du réacteur. Cette masse pèse 450 g. Par dosage à l'uréase, on constate qu'elle contient 13,5 g d'urée libre (soit 3% en poids) et 5,5 g d'acide sulfurique (soit 1,22%). Le rendement de condensation, exprimé en % d'urée est donc égal à:

$$\frac{60-13{,}5}{60} = 78\,\%$$

Comme le mélange contient 5,5 g d'acide sulfurique pur (56 mmoles) et 13,5 g d'urée libre soit 225 mmoles, on estime que le quart environ de l'urée non condensée est liée sous forme de sulfate, les 3/4 restant étant sous forme non salifiée (on estime en effet qu'une mole d'acide sulfurique fixe 1 mole d'urée).

On traite la moitié du produit (225 g) avec 500 ml d'éthanol dans un mixer pendant 3 minutes. On essore la suspension obtenue et on reprend les 225 g restant du produit avec l'alcool de lavage du premier traitement. L'ensemble du produit lavé, essoré est blanc et cristallin. Après séchage du produit pour éliminer l'alcool restant, on obtient 378 g d'une poudre blanche cristalline, non hygroscopique, contenant 0,56% d'urée libre (2,11 g) et 0,33% d'acide sulfurique (1,24 g) soit 35 mmoles d'urée non condensée et 12,6 mmoles d'acide sulfurique (mesure par alcalimétrie).

On voit donc que l'acide sulfurique résiduel doit être totalement combiné à l'urée qui est en excès, sous forme de sulfate d'urée. 85% de l'urée non condensée (225—35/225) et 77,5% de l'acide sulfurique (5,5—1,24/5,5) ont donc été éliminés au cours du lavage.

La teneur en azote du produit est de 5,41% (mesuré par analyse élémentaire), dont 0,25% correspond à l'urée non condensée et 5,16% à l'urée condensée. Ainsi, le poids de lactosylurée du mélange est donné par:

$[378-(2{,}11+1{,}24)]\cdot 5{,}16 = X\cdot 7{,}29$ (% de N de la lactosylurée)

$$\text{d'où, } X = \frac{374{,}65\times 5{,}16}{7{,}29} = 265{,}18\text{ g}$$

(poids total de lactosylurée), soit en % dans le mélange

$$\frac{265{,}18}{378} = 70{,}1\,\%$$

Rendement réel de la réaction:

$$\frac{265{,}18}{384} = 69\,\%$$

(384 = Pmol de la lactosylurée)

Le mélange obtenu consiste donc en:

265,18 g de lactosylurée, 119,47 g de lactose hydraté 2,11 g d'urée non condensée, en partie sous forme de sulfate, et 1,24 g d'acide sulfurique sous forme de sulfate d'urée.

Le produit cristallin se décompose entre 200 et 210° C, tandis que pour la lactosylurée pure on a: déc. 230—240° C).

En évaporant l'alcool de lavage, on peut récupérer un résidu solide constitué essentiellement d'urée (11,39 g) dont une partie (2,60 g) se trouve, sous forme de sulfate d'urée, combinée à 4,26 g d'acide sulfurique. Ce résidu peut être recyclé lors d'une fabrication ultérieure.

Exemple 2

On procède comme dans l'exemple 1, mais en remplaçant le lactose monohydrate cristallisé par du lactose monohydrate brut obtenu par concentration du lactosérum déprotéinisé. Ce lactose brut est préparé de la façon suivante:

1 litre de lactosérum déprotéinisé contenant du lactose à la concentration de 166 g/l et des sels minéraux et résidus de protéines à la concentration d'environ 34 g/l, est concentré sous pression réduite à 70° C au moyen d'un évaporateur rotatif jusqu'à un volume de 280 ml. Le concentrat est refroidi et ensemencé par quelques cristaux de lactose pur ce qui provoque, après repos, la cristallisation de 150 g de lactose relativement pur; cette quantité correspond à un rendement d'obtention du lactose de 90% environ.

Les eaux-mères, après évaporation de l'eau, fournissent un résidu épais contenant le reste du lactose (environ 16 g) et des sels minéraux et résidus protéiques (30 g). Après réaction de ce lactose purifié (360 g) avec 60 g d'urée comme dans l'exemple 1, on obtient 391 g d'un produit solide homogène de couleur noisette clair, contenant 4,83% en poids d'urée libre (18,8 g, 0,31 mole). La réaction de condensation a donc un rendement de 69% ce qui est un peu inférieur au rendement obtenu avec du lactose cristallisé.

On traite le produit avec de l'éthanol comme dans l'exemple 1 ce qui permet de réduire la teneur en urée libre de 4,83% à 2,5% en poids. Simultanément, la teneur en ions sulfates présents dans le mélange décroît de 1,22% à 0,69%.

## Exemple 3

On procède comme dans l'exemple 1 mais en remplaçant le lactose monohydrate crystallisé par du lactose monohydrate impur obtenu par évaporation à sec de lactosérum déprotéinisé. Le produit ainsi obtenu (mélange de lactose (83%), sels et résidus protéiques) est pâteux et hygroscopique. On conduit la réaction selon la méthode de l'exemple 1, en utilisant 433 g de ce mélange, soit 360 g de lactose. Le produit ainsi obtenu se présente comme une masse pâteuse à 7,4% d'urée libre mais en traitant celle-ci avec de l'éthanol comme décrit dans les exemplex précédents, puis en la séchant sous vide, elle se transforme en un produit solide cristallin. On obtient ainsi 385 g de produit contenant 2,5% d'urée libre et 0,67% d'ions sulfates. Si on tient compte que la masse réactionelle de départ contenait 13,85% d'urée libre, le rendement de la condensation est donné par

$$\frac{13{,}85 - 7{,}4}{13{,}85} = 46{,}57\,\%$$

ce qui est nettement inférieur aux rendements obtenus avec le lactose plus pur des exemples 1 et 2.

## Exemple 4

On mélange à température ambiante dans un réacteur 360 g de lactose monohydrate, 60 g d'urée et 100 ml d'une solution aqueuse contenant 5,5 g de $H_2SO_4$ concentré. On commence à chauffer et on porte graduellement la température à 110° C en 1 heure et demie. Le taux d'urée libre mesuré à ce moment là de 4,77%. On maintient la température à 110°C pendant 2 heures, après quoi le taux d'urée libre a passé à 2,34%. En continuant à chauffer à 110° C, le taux d'urée libre continue à diminuer, mais très lentement: 2,31% après 4 heures, 2,01% après 6 heures, 1,97% après 8 heures.

On a estimé que la réaction pouvait être arrêtée après deux heures à 110° C, car, après cette période, on a récupéré 410 g de produit sec contenant 9,4 g environ d'urée libre. D'où, rendement de la condensation (poids d'urée combinée/poids d'urée initial):

$$\frac{60 - 9{,}4}{60} = \frac{50{,}6}{60} = 85\,\%$$

Ce rendement est donc supérieur à celui (78%) obtenu après 23 heures à 70° C (voir exemple 1). On traite le mélange avec 1 litre d'éthanol dans un mixer, puis on sépare le solide lavé de couleur noisette par essorage. Après séchage on en obtient 370 g. Ce produit lavé et séché contient 1,68% d'urée libre. Le taux d'azote est de 6,35% ce qui correspond à un % d'urée total dans le produit (urée combinée + urée libre) de 14%.

Si on fait le rapport entre le taux d'urée combinée calculé par cette méthode et l'urée totale du produit on a:

$$\frac{14 - 1{,}68}{14} = \frac{12{,}32}{14} = 0{,}9$$

c'est-à-dire que le taux d'urée combinée est de 90% de l'urée totale. Ce résultat correspond approximativement au rendement calculé plus haut.

## Exemple 5

Préparation de lactosylurée en continu.

On mélange 360 g de lactose monohydrate (1 mole) avec 60 g d'urée (1 mole) et 10,6 ml d'une solution acide comprenant 100 ml d'eau et 6 ml d'acide sulfurique à 98%. On fait fondre ce mélange et on le coule en couche mince sur le tapis roulant d'un four tunnel. Ce four est équipé d'un système permettant, sur un premier tiers de sa longueur (zone I), de régler le chauffage selon un gradient de température et, dans les 2/3 restants (zone II), à une température constante. La zone I est soumise à un gradient de température allant de la température ambiante, à l'entrée, à une température de 100 à 110° C au bout de cette zone; la zone II est maintenue à la température constante de 100—110° C. Le mélange couché sur le tapis roulant pénètre donc dans le tunnel par la zone I et est porté progressivement, en 1 heure et demie, à 110° C après quoi, il entre dans la zone 2 où il reste de deux à trois heures à 110° C. A la sortie du tunnel le mélange est refroidi et se présente sous une forme cristalline légèrement mousseuse de couleur brun-clair, facile à détacher du tapis au moyen d'un conteau râcleur et se réduisant en poudre. On le recueille en continu et on en obtient 400 g environ.

La teneur totale en azote de cette poudre est de 7,44% et sa teneur en urée libre résiduelle est de 2,3% en poids, ce qui indique que 85% environ de l'urée s'est condensée et que 15% se trouve sous forme d'urée libre et de sulfate

d'urée. Bien que le taux de cette urée résiduelle libre ou salifiée (il reste 0,150 mole d'urée non réagie pour 0,112 mole d'acide sulfurique) soit assez bas pour ne constituer, avec les 15% de lactose n'ayant réagi (54,0 g), une impureté gênante lors de l'utilisation du produit comme additif alimentaire, on peut éliminer celle-ci en traitant la poudre dans un mixer en présence d'alcool selon le traitement indiqué dans l'exemple 1. Par cela on peut éliminer jusqu'à 80% de l'urée résiduelle qui n'est pas condensée.

Exemple 6

Dans un réacteur émaillé de 250 l, on dissout de l'urée, 37 kg (617 mole) dans 30 kg d'eau à 40° C environ. Puis, sous agitation, on ajoute 150 kg (408,3 mole) de lactose monohydrate à 98% et on chauffe à 80° C. Il se forme ainsi un mélange visqueux mais facilement mobile sons l'effet de l'agitateur. On abaisse alors le pH du mélange à 1,9—2 par adjonction, à 80° C, d'environ 8—10 kg d'une solution aqueuse de $H_2SO_4$ (1 volume d'acide pour 5 volumes d'eau), puis, progressivement au cours de la réaction, on ajoute d'autres portions d'acide dilué afin de continuellement maintenir le pH aux environs de 1,2—2. Pratiquement, on réalise cette addition progressive au moyen d'une pompe doseuse alimentée en acide dilué et asservie à un dispositif contrôlé par pH-mètre lequel provoque l'enclenchement et l'arrêt de la pompe suivant les variations du pH avec le temps. Le débit d'acide au cours de la réaction est d'environ 1 l/h. On suit la marche de la réaction par mesure du pouvoir rotatoire du mélange réactionnel au moyen d'un polarimètre usuel, la rotation spécifique du lactose pur étant de $|\alpha|_D = 52,4°$ (condition de départ). Prise, 2—4 g dissoute dans 40 ml $H_2O$; tube 200 mm. La rotation de lactosylurée n'étant que de 1,1°, celle-ci a été négligée dans les mesures de cinétiques ci-dessus. Après environ 6 heures à 80° C on avait ajouté environ 17 kg d'acide dilué (soit 4,5 kg de $H_2SO_4$ c'est-à-dire 1,92% en poids d'acide sur le total du mélange et 18% d'eau au total), et la mesure du pouvoir rotatoire indiquait un taux de conversion du lactose de 85%. On arrête la réaction et ajoute au mélange jaune-clair 40 kg de glace pilée. Après dissolution de cette glace sous agitation, on sépare, par centrifugation, le solide incolore qui s'est formé et on le lave avec 20 l d'eau glacée. Après essorage, on recueille 140 kg (81%) de lactosylurée monohydrate dont les propriétés, obtenues par analyse suivant les moyens habituels, sont les suivantes: %N trouvé : 6,73%; calculé : 7,28%. Urée résiduelle libre <0,3%. Acide sulfurique résiduel <0,1%. $|\alpha|_D^{20}$ +1,1°.F.240—245° C (dec.), début de brunissement vers 230° C.

On notera que, dans la forme de préparation ci-dessus, si la température excède notablement 80° C, la quantité d'acide à ajouter augmente du fait de la tendance du sel d'urée à former de l'ammoniac qui agit comme neutralisant de l'acidité. De plus, la présence d'une quantité d'eau trop importante conduit à une baisse du rendement de condensation en vertu du déplacement, vers la gauche de l'équilibre

$$\text{lactose} + \text{urée} \rightleftarrows \text{lactosylurée} + \text{eau}$$

Il est donc avantageux d'opérer en présence d'un minimum d'eau et d'acide avec contrôle du pH <2. Par ailleurs la présence d'un excès d'urée (1,5 équivalent pour 1 équivalent de lactose) favorise le rendement de conversion du lactose en lactosylurée; cependant, il devient alors utile de récupérer l'urée dissoute dans les eaux de lavage et de la recycler.

La lactosylurée obtenue sans purification ultérieure suivant le procédé ci-dessus n'est, contrairement aux produits correspondants de l'art antérieur, pas hygroscopique et se prête facilement à la fabrication, par mélange avec d'autres ingrédients (farines, premixes, tourteaux, arômes, etc ...), à la fabrication de fourrage pour ruminants d'excellente qualité.

**Revendications**

1. Procédé pour la préparation de lactosylurée de degré de pureté élevé propre à l'alimentation des ruminants en azote suivant lequel on condense le lactose et l'urée en présence d'eau et d'un catalyseur acide, caractérisé par le fait qu'on opère la condensation entre 70 et 110° C avec une quantité d'eau minimum quoique suffisante pour que la consistance du mélange réactionnel reste fluide sous agitation mais soit également assez ferme pour qu'on puisse déposer ledit mélange sans qu'il coule sur une surface plane horizontale.

2. Procédé suivant la revendication 1, caractérisé par le fait qu'en cours de réaction, on élimine progressivement l'eau par évaporation.

3. Procédé suivant la revendication 1, caractérisé par le fait qu'après la réaction de condensation, on traite la masse réactionnelle par de l'eau ou un solvant hydrophile froid puis qu'on sépare le solide cristallisé du liquide, de manière que les produits n'ayant pas réagi soient entraînés, à l'état dissous dans ledit liquide, et qu'on recueille ainsi une lactosylurée solide de très haut degré de pureté.

4. Procédé suivant la revendication 3, caractérisé par le fait qu'on mélange le produit de réaction brut à de l'eau glacée ou de la glace, qu'on agite fortement et qu'on sépare le solide du liquide par essorage ou centrifugation.

5. Procédé suivant la revendication 3, caractérisé par le fait qu'on broie et triture la masse réactionelle brute avec de l'éthanol ce qui a pour effet de sélectivement dissoudre l'urée non transformée, puis qu'on sépare le solide purifié de l'éthanol par essorage.

6. Procédé suivant la revendication 1, caractérisé par le fait que l'acide est du $H_2SO_4$, du HCl ou du $H_3PO_4$ dont la quantité est de 0,5 à 2,5% en poids du mélange réactionnel.

7. Procédé suivant la revendication 1, caractérisé par le fait qu'on chauffe progressivement jusqu'à 110°C puis qu'on maintient cette température quelques heures sous pression ordinaire ou sous pression réduite.

8. Procédé suivant les revendications 1 ou 7, caractérisé par le fait qu'on travaille en continu dans un four tunnel, la masse réactionnelle étant étalée en couche sur un tapis roulant.

**Patentansprüche**

1. Verfahren zur Herstellung einer Lactose-Harnstoffverbindung von hoher Reinheit, geeignet für die Ernährung von Wiederkäuern mit Stickstoff, wonach man die Laktose und den Harnstoff in Gegenwart von Wasser und einem sauren Katalysator kondensiert, dadurch gekennzeichnet, daß man die Kondensierung zwischen 70 und 110° mit einer Geringstwassermenge durchführt, die ausreichend ist, damit die Konsistenz der Reaktionsmischung unter Rühren flüssig bleibt, jedoch fest genug ist, damit man diese Mischung ablagern kann, ohne daß sie auf einer ebenen, waagerechten Oberfläche wegfließt.

2. Verfahren nach Anspruch 1, dadurch gekennzeichnet, daß man im Verlauf der Reaktion schrittweise das Wasser mittels Verdampfung entfernt.

3. Verfahren nach Anspruch 1, dadurch gekennzeichnet, daß man nach der Kondensierungsreaktion die Reaktionsmasse mit Wasser oder einem kalten hydrophilen Lösungsmittel behandelt, daß man dann den kristallisierten Feststoff der Flüssigkeit in einer Weise abtrennt, daß die Produkte, die nicht reagiert haben, in gelöstem Zustand in der Flüssigkeit mitgenommen werden, und daß man so eine feste Laktose-Harnstoffverbindung mit einem sehr hohen Reinheitsgrad gewinnt.

4. Verfahren nach Anspruch 3, dadurch gekennzeichnet, daß man das Ausgangsreaktionsprodukt mit Eiswasser oder Eis mischt, daß man stark rührt und daß man den Feststoff der Flüssigkeit mittels Absaugung oder Zentrifugierung abtrennt.

5. Verfahren nach Anspruch 3, dadurch gekennzeichnet, daß man die Ausgangsreaktionsmasse mit Äthanol zerkleinert und feinmahlt, was zur Folge hat, daß der nicht umgewandelte Harnstoff selektiv aufgelöst wird, und daß man dann den gereinigten Feststoff vom Äthanol durch Absaugen abtrennt.

6. Verfahren nach Anspruch 1, dadurch gekennzeichnet, daß die Säure $H_2SO_4$, HCl oder $H_3PO_4$ mit einer Menge von 0,5 bis 2,5 Gew.-% der Reaktionsmischung ist.

7. Verfahren nach Anspruch 1, dadurch gekennzeichnet, daß man schrittweise bis auf 110° erhitzt und daß man dann diese Temperatur einige Stunden lang bei normalen Druck oder bei vermindertem Druck hält.

8. Verfahren nach Anspruch 1 oder 7, dadurch gekennzeichnet, daß man kontinuierlich in einem Tunnelofen arbeitet, wobei die Reaktionsmasse als Schicht auf einem Laufband ausgebreitet ist.

**Claims**

1. A process for the preparation of high purity lactosylurea suitable for supplementing nitrogen to ruminants according to which one condenses lactose and urea in the presence of water and an acid catalyst, characterized by the fact that one performs the condensation between 70 and 110° with quantity of water minimum although sufficient for the reaction mixture to stay fluid enough under stirring but also stiff enough for enabling it to be spread on a horizontal planar surface without collapsing.

2. A process according to claim 1, characterized by the fact that the water is progressively eliminated by evaporation in the course of the reaction.

3. A process according to claim 1, characterized by the fact that, after the reaction of condensation, one treats the reaction mass with water or a cold hydrophilic solvent then one separates the resulting crystallized solid from the liquid so that the still unreacted products are carried away in the dissolved state in said liquid and one thus collects a solid lactosylurea of very high degree of purity.

4. A process according to claim 3, characterized by the fact that one mixes the crude reaction product with ice or icewater, one agitates strongly the mixture and one separates the solid from the liquid by draining or centrifugation.

5. A process according to claim 3, characterized by the fact that one crushes and grinds the crude reaction mass with ethanol which causes the selective dissolution of the untransformed urea, then one separates the purified solid from the ethanol by draining.

6. A process according to claim 1, characterized by the fact that the acid is $H_2SO_4$, HCl or $H_3PO_4$ the quantity of which amounts to 0.5 to 2.5% by weight of the reaction mixture.

7. A process according to claim 1, characterized by the fact that one progressively heats the mixture to 110°C then one maintains this temperature for several hours under ordinary or reduced pressure.

8. A process according to claims 1 or 7, characterized by the fact that one operates in a continuous manner in a tunnel-oven, the reaction mass being spread as a continuous layer on a moving belt.